# EUROPÄISCHE PATENTANMELDUNG

(11) **EP 4 461 320 A1**
(43) Veröffentlichungstag der Anmeldung: **13.11.2024**
(21) Anmeldenummer: 23187977.6
(22) Anmeldetag: 27.07.2023
(51) Int. Cl.: A61L 2/04

(54) **VERFAHREN UND VORRICHTUNG ZUM STERILISIEREN VON ROHMATERIAL ZUM HERSTELLEN ZUMINDEST EINES TEILS EINES FASERN UMFASSENDEN BEHÄLTERS, VERFAHREN ZUM HERSTELLEN EINES FASERN UMFASSENDEN BEHÄLTERS UNTER VERWENDUNG DES STERILISIERTEN ROHMATERIALS UND BEHÄLTERBEHANDLUNGSANLAGE**

(30) Priorität: 20.12.2022 DE 102022134088
(71) Anmelder: KRONES Aktiengesellschaft, 93073 Neutraubling (DE)
(72) Erfinder: ZACHARIAS, Dr. Joerg, Neutraubling (DE); WINZINGER, Frank, Neutraubling (DE)
(74) Vertreter: Grünecker Patent- und Rechtsanwälte PartG mbB

(57) **Zusammenfassung**

Die Erfindung betrifft ein Verfahren zum Sterilisieren von Rohmaterial (3, 13, 21) zum Herstellen zumindest eines Teils eines Fasern umfassenden Behälters, wobei das Verfahren umfasst: ein Bereitstellen des Rohmaterials (3, 13, 21) und ein Sterilisieren des Rohmaterials (3, 13, 21) zu sterilisiertem Rohmaterial. Weiter betrifft die Erfindung sterilisiertes Rohmaterial zum Herstellen zumindest eines Teils eines Fasern umfassenden Behälters, wobei das sterilisierte Rohmaterial mittels des Verfahrens erhalten wurde, ein Verfahren zum Herstellen eines Fasern umfassenden Behälters unter Verwendung des sterilisierten Rohmaterials und den Fasern umfassenden Behälter. Zudem betrifft die Erfindung eine Vorrichtung zum Sterilisieren von Rohmaterial zum Herstellen zumindest eines Teils eines Fasern umfassenden Behälters, wobei die Vorrichtung ausgebildet ist, das Verfahren zum Sterilisieren von Rohmaterial (3, 13, 21) auszuführen.

## Beschreibung

Die Erfindung betrifft ein Verfahren und eine Vorrichtung zum Sterilisieren von Rohmaterial zum Herstellen zumindest eines Teils eines Fasern umfassenden Behälters, ein sterilisiertes Rohmaterial, ein Verfahren zum Herstellen eines Fasern umfassenden Behälters unter Verwendung des sterilisierten Rohmaterials, einen Fasern umfassenden Behälter und eine Behälterbehandlungsanlage gemäß den unabhängigen Ansprüchen.

### Stand der Technik

Für die Herstellung von Fasern umfassenden Behältern ist es bekannt, Zellulosefasern oder Holzfasern oder dergleichen zu verwenden. Diese Fasern können zusammen mit einem Fluid als Pulpe zur Verfügung stehen. Je nach Herkunft der Fasern und beispielsweise des Fluids kann eine mikrobiologische Keimbelastung vorliegen, beispielsweise durch Bakterien, Schimmelpilze, Hefen und/oder Sporen. Die mikrobiologische Keimbelastung kann ein in den Behälter abgefülltes Produkt negativ beeinflussen.

### Aufgabe

Die Aufgabe der Erfindung ist es, ein Verfahren und eine Vorrichtung zum Sterilisieren von Rohmaterial zum Herstellen zumindest eines Teils Fasern umfassenden Behälters, ein sterilisiertes Rohmaterial, ein Verfahren zum Herstellen eines Fasern umfassenden Behälters unter Verwendung des sterilisierten Rohmaterials, einen Fasern umfassenden Behälter und eine Behälterbehandlungsanlage zur Verfügung zu stellen, die eine mikrobiologische Belastung reduzieren können.

### Lösung

Die Aufgabe wird gelöst durch das Verfahren und die Vorrichtung zum Sterilisieren von Rohmaterial zum Herstellen zumindest eines Teils eines Fasern umfassenden Behälters, das sterilisierte Rohmaterial, das Verfahren zum Herstellen eines Fasern umfassenden Behälters unter Verwendung des sterilisierten Rohmaterials, den Fasern umfassenden Behälter und die Behälterbehandlungsanlage gemäß den unabhängigen Ansprüchen. Weitere Ausführungsformen sind in den Unteransprüchen offenbart.

Das erfindungsgemäße Verfahren zum Sterilisieren von Rohmaterial zum Herstellen zumindest eines Teils eines Fasern umfassenden Behälters umfasst ein Bereitstellen des Rohmaterials und ein Sterilisieren des Rohmaterials zu sterilisiertem Rohmaterial.

Das Rohmaterial kann also vor dem Herstellen zumindest eines Teils eines Fasern umfassenden Behälters sterilisiert werden. Ein Sterilisieren des Teils des Fasern umfassenden Behälters oder des ganzen Behälters nach dem Herstellen ist dann in bestimmten Fällen nicht erforderlich. Alternativ kann aber auch ein zusätzliches Sterilisieren nach dem Herstellen erfolgen.

Das Rohmaterial umfasst die Fasern und kann auch weitere Stoffe, wie Wasser oder Zusätze, umfassen. Das Rohmaterial kann als Pulpe zur Verfügung gestellt werden. So kann die Pulpe vor der Verwendung für die Herstellung eines Fasern umfassenden Behälters sterilisiert werden.

Die Fasern können Lignin, Bananenblätter und/oder Chinin umfassen. Die Fasern können beispielsweise Zellulosefasern, Fasern von Nadelhölzern, Blattgehölzen und/oder Platanen und/oder von Gräsern, Schilf und/oder Bambus oder dergleichen umfassen. Die Fasern können Seidenfäden, Spinnenfäden, Algen, natürliche Fasern (wie Donau-Silphie-Fasern, Hanf, Mais, Baumwolle), Bananenschalen, Orangenschalen, Gras, Stroh, Kartoffelstärke oder aufbereiteten Kuhdung umfassen. Auch können Zellstofffasern vorgesehen sein, die einem Prozess entstammen, durch den sie künstlich gezüchtet wurden. Diese alternativen Materialien können bei Materialengpässen von Holz als Grundstoff für eine fluide Masse mit Fasern, den Grundstoff vollständig oder teilweise ersetzen.

Die Fasern können Fasermischungen aus holzfremden Material, beispielsweise Baumwolle, Hanf, und/oder Textilfasern umfassen.

Beispielsweise können die Fasern Viskosefasern umfassen. Eine Viskosefaser ist eine künstliche Faser aus regenerierter Zellulose, wobei die Viskosefasern als Ausgangsbasis 100% Zellulose haben, die in einem mehrstufigen Verfahren behandelt wird. Die Viskosefasern können eine flache oder kabelförmige Struktur, eine trilobale Form oder eine doppelt trilobale Struktur umfassen. Die Fasern können eine flächige oder hohle Struktur oder eine geriffelte oder raue äußere Oberfläche umfassen.

Es können auch verschiedenartige Fasern in Kombination verwendet werden. Beispielsweise können Fasern mit unterschiedlichen äußeren Oberflächen und/oder Formen und/oder hohler Struktur und/oder flächiger Struktur und/oder aus verschiedenen Materialien kombiniert werden. Auch unterschiedliche Längenverteilungen der Fasern können dabei vorliegen.

Durch den natürlichen Ursprung der Fasern können diese biologisch abbaubar sein. Zudem sind sie nachhaltig und nachwachsend.

Die Fasern können in einer Pulpe verwendet werden. Die Pulpe kann eine Mischung aus Wasser, beispielsweise mit Additiven, und Fasern sein oder umfassen.

Das Rohmaterial kann Ballenware, Sackware, recyceltes Papier, Kartonagen oder auch vordispergiertes Rohmaterial (jeweils in zerkleinertem Zustand) umfassen, das eine mikrobiologische Grundbelastung mitbringen kann, die ein später in einen das Rohmaterial umfassenden Behälter eingefülltes Produkt verderben kann.

Das Rohmaterial kann auch recyceltes Rohmaterial sein oder umfassen. Das recycelte Rohmaterial kann sterilisiertes Rohmaterial sein, das bereits verwendet wurde, beispielsweise Reste aus einem Herstellungsprozess von Fasern umfassenden Behältern und/oder recycelte Fasern umfassende Behälter, die sterilisiertes Rohmaterial umfassten.

Daher ist ein Sterilisieren des Rohmaterials zum Herstellen zumindest eines Teils eines Fasern umfassenden Behälters sinnvoll. Eine Hitzeeinwirkung auf den Fasern umfassenden Behälter während eines Trocknungsprozesses nach dem Herstellen kann nicht ausreichend sein hinsichtlich einer Keimabtötung.

Das Sterilisieren kann ein physikalisches und/oder elektrothermisches Sterilisieren umfassen. Beispielsweise kann das physikalische Sterilisieren mittels eines physikalischen Sterilisators erfolgen, wie mittels Mikrowellenstrahlung, mittels eines Ohmschen Erhitzers, mittels Induktion, mittels Ultraviolettstrahlung, mittels Infrarotlicht, mittels einer Hochfrequenzerhitzung, mittels eines Pulsed Electric Field, mittels Plasma und/oder mittels Hochdruck.

Bei dieser Art der Sterilisierung ist es nicht erforderlich, dass zusätzliches Wasser in das Rohmaterial eingetragen wird. Das physikalische und/oder elektrothermische Sterilisieren kann für hochviskoses Rohmaterial verwendet werden.

Das physikalische Sterilisieren kann auf einem Transferweg des Rohmaterials zur Formmaschine erfolgen, beispielsweise direkt in einem Zuleitungssystem und/oder in einem dafür vorgesehenen Aggregat, Ausmischbehälter oder allgemein in einem dafür vorgesehenen Tank.

Das Sterilisieren kann alternativ oder zusätzlich eine thermische Erhitzung umfassen. Beispielsweise kann die thermische Erhitzung mittels einer Kurzeiterhitzungsvorrichtung erfolgen. Das Rohmaterial kann durch die Kurzeiterhitzungsvorrichtung gepumpt und in ihr sterilisiert werden, bevor es in die Form gelangen kann. Unter Pumpen kann auch ein Leiten mittels Überdruck oder Gravitation verstanden werden. Die Kurzeiterhitzungsvorrichtung kann beispielsweise einen Röhrenwärmetauscher (das kann Fasern des Rohmaterials schonen) umfassen, der entsprechend der Viskosität des Rohmaterials und dem für das Rohmaterial notwendigen Wärmeübergang ausgelegt sein kann, sodass eine entsprechende Keimreduzierung erreicht werden kann oder die Kurzeiterhitzungsvorrichtung kann einen Plattenwärmetauscher umfassen. Das Rohmaterial kann durch die Kurzeiterhitzungsvorrichtung mindestens auf eine Temperatur von 72°C für mindestens etwa 15 bis 30 Sekunden gebracht werden. Bei verwandten Anwendungen bei Getränken werden diese moderaten Bedingungen (Temperatur von 72°) auch als Pasteurisation bezeichnet. Daher wird dieser Begriff hier auch im Zusammenhang mit dem Rohmaterial eingeführt, da die Pasteurisation auch eine Keimreduzierung durch diese und vergleichbare Verfahren beschreiben kann. Danach kann das sterilisierte Rohmaterial regenerativ abgekühlt werden. Die Kurzeiterhitzungsvorrichtung kann die gleiche Wärmequelle verwenden wie auch diejenige Kurzeiterhitzungsvorrichtung, die zum Sterilisieren des abzufüllenden Produkts vorgesehen ist. Auch kann auf die Wärmerückgewinnung und/oder Rekuperation und/oder Anbindung an andere Wärmequellen und Wärmeverbraucher beim Betrieb geachtet werden. Alternativ kann eine thermische Erhitzung ein Erhitzen des Rohmaterials über mehrere Stunden umfassen.

Anders gesagt kann das Sterilisieren durch einen indirekten Wärmeeintrag in das Rohmaterial erfolgen.

Beispielsweise kann das Sterilisieren in einem Wärmetauscher erfolgen, beispielsweise in einem Röhrenwärmetauscher, wobei aber auch andere Wärmetauscherbauarten in Frage kommen können, wie z.B. ein Plattenwärmetauscher oder ein Schabewärmetauscher.

Beispielsweise kann eine Heißhaltestrecke in Form einer Leitung vorgesehen sein, in welcher eine vorbestimmte Temperatur des Rohmaterials gehalten werden kann, bevor es wieder abkühlt werden kann. Es ist nicht erforderlich, dass das Rohmaterial nach der Heißhaltestrecke abgekühlt wird. Ohne Abkühlung kann beispielsweise die Sterilität des Rohmaterials erhalten blieben bis es einen aseptischen Raum erreicht, der beispielsweise weiter unten im Zusammenhang mit der Behälterbehandlungsanlage beschrieben wird.

Beispielsweise kann das Abkühlen regenerativ erfolgen, indem Wärme aus dem Rohmaterial an ein Wärmeaustauschmedium, welches wieder für ein Erwärmen an anderer Stelle verwendet werden kann, beispielsweise von neuen, zu sterilisierendem Rohmaterial, oder indem Wärme an neues, zu sterilisierendes Rohmaterial abgegeben werden kann. Beispielsweise kann dies durch einen sogenannten Produkt-Produkt-Wärmetauscher(-abschnitt) erfolgen.

Beispielsweise kann eine Erhitzung des Rohmaterials auf mindestens 75°C, beispielsweise auf mindestens 90°C, erfolgen. Es kann auch eine Erhitzung des Rohmaterials auf mindestens 110°C erfolgen.

Beispielsweise kann eine Erhitzung des Rohmaterials auf die obenstehenden Temperaturen dann jeweils mindestens für 15 Sekunden, beispielsweise für jeweils mindestens 30 Sekunden, erfolgen. Es kann auch sein, dass größere Zeitdauern von mehr als 60 Sekunden vorgesehen sind.

Durch unterschiedlich intensive Hitzebelastungen, die bei Getränken den Zusammenhang von Temperaturprofil und Einwirkdauer beschreiben kann, kann eine unterschiedlich effektive Keimreduzierung erreicht werden. Daher wird hier angemerkt, dass dies bei der Auswahl der Bedingungen berücksichtigt werden kann. Die Abhängigkeit hierzu können die zu erwartenden Keime bzw. mikrobiologische Belastung vorgeben, die je nach Ursprung des Rohmaterials und/oder der Region unterschiedlich sein können. In der Regel spricht man von moderaten Reduzierungen, wie man sie aus aseptischen Anwendungen der Getränkeindustrie kennt, in Größenordnungen der Reduzierung zwischen 4 und 6 Log-Stufen. Man könnte das auch als Pasteurisation des Rohmaterials bezeichnen. Von Sterilisationsbedingungen kann man bei Log-Stufen in der Größenordnung von 9-12 Log-Stufen sprechen, wie sie beispielsweise bei der Produktion von Sterilwasser erreicht werden können.

Energie, die zum Erhitzen des Rohmaterials verwendet wird, kann für weitere Behandlungsschritte beim Herstellen eines Fasern umfassenden Behälters basierend auf dem Rohmaterial, verwendet werden. Beispielsweise für eine Trocknung und/oder ein Erhitzen der Form über einen Gas-Wasser-Wärmetauscher und/oder für ein Erhitzen des Formmediums (beispielsweise mittels Wasser oder Luft).

Das Sterilisieren kann alternativ oder zusätzlich ein chemisches Sterilisieren umfassen. Beispielsweise kann das chemische Sterilisieren mittels einer Zugabe von chemischem Desinfektionsmittel erfolgen. Beispielsweise kann das chemische Desinfektionsmittel H₂O₂, O₂, O₃, NaClO₂, NaClO, HClO, HOCl-Wasser oder elektrochemische aktiviertes Wasser umfassen.

Beim chemischen Sterilisieren kann das Rohmaterial direkt sterilisiert werden. Das chemische Desinfektionsmittel kann einer Dispergierflüssigkeit in entsprechender Konzentration zugegeben werden und je nach Konzentration und Einwirkzeit wirken. Das chemische Sterilisieren kann bei einem Ausmischvorgang des Rohmaterials erfolgen.

Das Rohmaterial kann Fasern umfassen. Beispielsweise können es trockene Fasern sein. Beispielsweise können die Fasern Naturfasern umfassen. Bei trockenen Fasern kann eine Sterilisierung beispielsweise mittels Heißluft und/oder Ultraviolettstrahlung erfolgen.

Das Rohmaterial kann Fasern und ein Fluid umfassen. Die Mischung aus Fasern und Fluid kann eine Pulpe darstellen. Beispielsweise können die Fasern Naturfasern umfassen. Beispielsweise kann das Fluid Wasser umfassen, wobei beispielsweise das Fluid Additive umfassen kann.

Weiter ist ein sterilisiertes Rohmaterial zum Herstellen zumindest eines Teils eines Fasern umfassenden Behälters vorgesehen, wobei das sterilisierte Rohmaterial mittels des Verfahrens zum Sterilisieren von Rohmaterial zum Herstellen zumindest eines Teils eines Fasern umfassenden Behälters, wie oben oder weiter unten beschrieben, erhalten wurde.

Mittels des sterilisierten Rohmaterials kann ein Deckel für einen Behälter, beispielsweise einen Fasern umfassenden Behälter, wie oben oder weiter unten beschrieben, hergestellt werden.

Weiter ist ein Verfahren zum Herstellen eines Fasern umfassenden Behälters unter Verwendung des sterilisierten Rohmaterials, wie oben oder weiter unten beschrieben, vorgesehen. Das sterilisierte Rohmaterial kann ein eine Form eingebracht und dort gepresst werden zum Ausformen des Fasern umfassenden Behälters.

Zudem ist ein Fasern umfassender Behälter vorgesehen, wobei zumindest ein Teil des Fasern umfassenden Behälters sterilisiertes Rohmaterial umfasst, das mittels des Verfahrens, wie oben oder weiter unten beschrieben, erhalten wurde.

Der Fasern umfassende Behälter kann mittels des Verfahrens zum Herstellen eines Fasern umfassenden Behälters unter Verwendung des sterilisierten Rohmaterials, wie oben oder weiter unten beschrieben, hergestellt worden sein. Der Behälter kann einen Deckel umfassen.

Weiter ist eine Vorrichtung zum Sterilisieren von Rohmaterial zum Herstellen zumindest eines Teils eines Fasern umfassenden Behälters vorgesehen, wobei die Vorrichtung ausgebildet ist, das Verfahren zum Sterilisieren von Rohmaterial zum Herstellen zumindest eines Teils eines Fasern umfassenden Behälters, wie oben oder weiter unten beschrieben, auszuführen.

Die Vorrichtung kann weiter eine Zuführvorrichtung für Rohmaterial und eine Sterilisiervorrichtung zum Sterilisieren des Rohmaterials umfassen. Die Zuführvorrichtung kann einen Ausmischbehälter umfassen, beispielsweise zur Aufnahme des Rohmaterials. Das Rohmaterial kann in dem Ausmischbehälter dispergiert werden.

Die Sterilisiervorrichtung kann mindestens eines der Folgenden umfassen:
- eine Vorrichtung zur Erzeugung von Mikrowellenstrahlung, einen Ohmschen Erhitzer, eine Vorrichtung zur Induktionserhitzung, eine Vorrichtung zur Erzeugung von Ultraviolettstrahlung, eine Vorrichtung zur Erzeugung von Infrarotlicht, eine Vorrichtung zur Erzeugung einer Hochfrequenz, eine Vorrichtung zur Erzeugung eines Pulsed Electric Field, eine Vorrichtung zur Erzeugung von Plasma, eine Vorrichtung zur Erzeugung von Hochdruck, eine Kurzeiterhitzungsvorrichtung, einen Wärmetauscher, eine Zugabevorrichtung zum Zugeben von chemischem Desinfektionsmittel.

Besteht das Rohmaterial nur aus trockenen Bestandteilen, so kann eine Sterilisation mittels heißem Gas, beispielsweise Luft, erfolgen. In dem Fall kann die Sterilisationsvorrichtung ein Gebläse umfassen. Auch ein Gasbrenner oder eine Heizung oder dgl. kann Bestandteil der Sterilisationsvorrichtung sein.

Es kann sein, dass eine Herstellung des Behälters sowohl aus sterilisiertem als auch aus nicht sterilisiertem Rohmaterial erfolgen kann. Beispielsweise kann eine äußere Schicht des Behälters nicht sterilisiertes Material und eine weiter innen liegende Schicht des Behälters sterilisiertes Material umfassen. Beispielsweise kann die innere Schicht aus sterilisiertem Material bestehen. Die innere Schicht kann beispielsweise mit in den Behälter abgefülltem Produkt in Kontakt kommen. Die Herstellung des Behälters kann beispielsweise derart erfolgen, dass in eine Form zuerst nicht sterilisiertes Material eingeführt wird und anschließend zur Bildung einer weiter innenliegenden Schicht sterilisiertes Rohmaterial. Zwischen den beiden Schritten kann eine zumindest teilweise Trocknung und/oder Verfestigung der äußeren Schicht erfolgen.

Es kann sein, dass der Behälter nach seiner Formung unmittelbar einer Abfüllung und einem Verschließen mit einem Verschluss unterzogen werden kann. Anschließend können mehrere Behälter zusammen als Gebinde verpackt und/oder auf einer Palette aufgestapelt werden.

Allgemein ist auch daran gedacht, das Rohmaterial zu entgasen. So kann zwischen dem Ausmischbehälter und der Form, beispielsweise zwischen Ausmischbehälter und dem Wärmetauscher, beispielsweise auch zwischen zwei Sektionen des Wärmetauschers für ein vorgewärmtes Rohmaterial, ein Entgasungsbehälter angeordnet sein, durch den das Rohmaterial befördert werden kann und in dem beispielsweise ein Unterdruck anliegen kann. Alternativ oder zusätzlich kann das Wasser entgast werden, bevor es in den Ausmischbehälter kommt. Es kann sein, dass das Wasser oder das Rohmaterial vor der Entgasung noch erwärmt wird, beispielsweise auf eine Temperatur im Bereich zwischen 30°C und 80°C, beispielsweise zwischen 50°C und 70°C. Die Bereichsgrenzen des Wertebereichs können miteingeschlossen sein. Beispielsweise kann durch eine Entgasung Sauerstoff aus dem Material entfernt werden, der später nach der Abfüllung des Behälters schädlich für das Getränk sein kann. Der Entgasungsbehälter kann beispielsweise an eine Vakuumpumpe angeschlossen sein. Im Zulauf des Entgasungsbehälters kann ein Ventil vorhanden sein. Bei der Entgasung kann es sich auch um eine unabhängige Erfindung handeln.

Weiter ist eine Behälterbehandlungsanlage vorgesehen, die eine Bereitstellungsvorrichtung zum Bereitstellen einer sterilen, Fasern umfassenden Pulpe, eine Herstellungsvorrichtung zum Herstellen eines die Fasern umfassenden Behälters aus der sterilen, Fasern umfassenden Pulpe und eine Füll- und Verschließvorrichtung zum Füllen des sterilen, Fasern umfassenden Behälters mit Produkt und zum Verschließen des befüllten sterilen, Fasern umfassenden Behälters und mindestens einen aseptischen Raum umfasst, der ausgebildet ist, eine definierte aseptische Umgebungsbedingung für den sterilen, Fasern umfassenden Behälter aufrecht zu erhalten, und der sich von der Bereitstellungsvorrichtung bis zu der Füll- und Verschließvorrichtung erstreckt.

Der mindestens eine aseptische Raum kann mit gefilterter Luft und/oder mit steriler Luft beaufschlagt werden.

Der mindestens eine aseptische Raum kann derart ausgebildet sein, dass die Sterilität von sterilen Elementen der Behälterbehandlungsanlage, wie Maschinen oder verwendete Materialien, wie beispielsweise die sterile, Fasern umfassende Pulpe, im aseptischen Raum erhalten bleiben kann.

Beispielsweise kann der mindestens eine aseptische Raum ein Raum mit kontaminationsarmer Umgebung sein.

Die kontaminationsarme Umgebung, bei der es sich beispielsweise um einen Reinraum handeln kann, kann durch eine oder Kombinationen der folgenden Maßnahmen erhalten werden:
- Die kontaminationsarme Umgebung (beispielsweise innerhalb eines Maschinenschutzes) kann unter einen Überdruck im Vergleich zur Umgebungsatmosphäre gesetzt werden, indem beispielsweise durch Feinfilter filtrierte Luft zumindest während eines Betriebsmodus' der Behälterbehandlungsanlage (beispielsweise einer Produktion) in den aseptischen Raum eingeblasen werden kann.
- Antriebe für verschiedenste Bewegungen einer Form zum Herstellen des Fasern umfassenden Behälters (beispielsweise eine Anschwemmform oder ein Formträger) und/oder von Transportelementen können außerhalb des aseptischen Raums angeordnet sein. Beispielsweise können die Antriebe für ein Öffnen und Schließen der Form (Kavität) und/oder für ein Anheben oder Senken eines Fasern umfassenden Behälters und/oder eine Bewegung einer Fülldüse außerhalb des aseptischen Raums angeordnet sein.
- Eine Oberfläche einer inneren Wandung des aseptischen Raums kann in regelmäßigen Abständen gereinigt und/oder sterilisiert werden. Beispielsweise können die Reinigung und/oder das Sterilisation durch ein Bespritzen oder Bedampfen mit Lauge, Säure, Entkeimungsflüssigkeit, Wasserstoffperoxid (gasförmig oder flüssig), Reinigungsschaum oder dergleichen erfolgen. Beispielsweise können bei der Reinigung und/oder Sterilisation die Innen- und Außenseiten der Form und/oder der Fülldüsen und/oder von Verschließköpfen miteinbezogen werden. Beispielsweise können auch Oberflächen von inneren Wandungen des Maschinenschutzes einbezogen werden.
- Die kontaminationsarme Umgebung kann gegen die Umgebungsatmosphäre abgedichtet werden. Bei einem Rundläufer (mehrere Formen und/oder Fülldüsen und/oder Verschließköpfe jeweils oder gemeinsam auf einem Karussell) kann die Dichtung als ein Wasserschloss oder eine Gummidichtung ausgebildet sein, das/die den drehenden Teil zum stehenden Teil des Rundläufers abdichten kann.

Die Behälterbehandlungsanlage kann zudem eine CIP/SIP-Anlage (Clean-in-Place/Sterilisationin-Place-Anlage) für die Formen zur Herstellung der Fasern umfassenden Behälter umfassen.

Die CIP/SIP-Anlage kann für Medien, wie Lauge, Wasser, Säure und/oder Additive, jeweils mindestens einen Tank zum Bereitstellen eines der Medien umfassen.

Im Tank kann ein Wärmetauscher vorgesehen sein, beispielsweise zum Erhitzen eines der im Tank bereitgestellten Medien vor seiner Verwendung.

Die CIP/SIP-Anlage kann einen separaten Kreislauf für ein Leitungssystem, in dem die fasern umfassende Pulpe transportiert werden kann, umfassen. Beispielsweise kann die CIP/SIP-Anlage einen weiteren separaten Kreislauf für ein Leitungssystem, in dem Produkt transportiert werden kann, umfassen.

Es kann vorgesehen sein, dass die Behälterbehandlungsanlage eine separate CIP/SIP-Anlage für ein Leitungssystem, in dem die fasern umfassende Pulpe transportiert werden kann, umfasst.

Ein Reinigen/Sterilisieren des bzw. der zuvor erwähnten Leitungssysteme kann bei einer Temperatur von mindestens 60 °C oder mindestens 80 °C oder mindestens 120 °C erfolgen.

### Kurze Figurenbeschreibung

Die beigefügten Figuren stellen beispielhaft zum besseren Verständnis und zur Veranschaulichung Aspekte und/oder Ausführungsbeispiele der Erfindung dar. Es zeigt:
Figur 1 eine erste Ausführungsform einer Vorrichtung zum Sterilisieren von Rohmaterial zum Herstellen zumindest eines Teils eines Fasern umfassenden Behälters,
Figur 2 eine zweite Ausführungsform einer Vorrichtung zum Sterilisieren von Rohmaterial zum Herstellen zumindest eines Teils eines Fasern umfassenden Behälters,
Figur 3 eine dritte Ausführungsform einer Vorrichtung zum Sterilisieren von Rohmaterial zum Herstellen zumindest eines Teils eines Fasern umfassenden Behälters und
Figur 4 eine Behälterbehandlungsanlage.

### Ausführliche Figurenbeschreibung

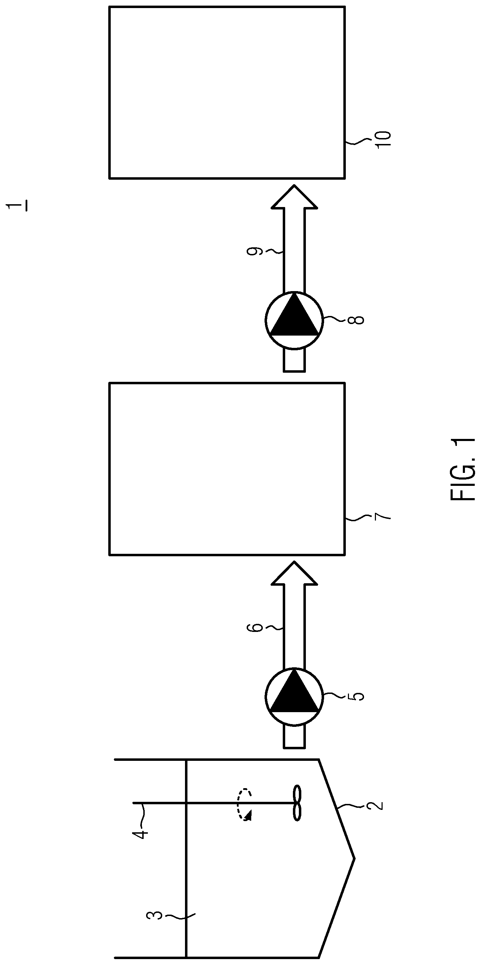

Die Figur 1 zeigt eine erste Ausführungsform einer Vorrichtung 1 zum Sterilisieren von Rohmaterial 3 zum Herstellen zumindest eines Teils eines Fasern umfassenden Behälters. Das zu sterilisierende Rohmaterial 3 wird in einem Ausmischbehälter 2 bereitgestellt, in dem es mittels eines Rührers 4 dispergiert werden kann. Danach wird das Rohmaterial 3 mittels einer ersten Pumpe 5 durch eine erste Rohrleitung 6 zu und durch eine Kurzzeiterhitzungsvorrichtung 7 transportiert. Die Kurzzeiterhitzungsvorrichtung 7 kann einen Plattenwärmetauscher oder einen Röhrenwärmetauscher umfassen. In der Kurzzeiterhitzungsvorrichtung 7 wird das Rohmaterial zu sterilisiertem Rohmaterial sterilisiert, beispielsweise bei einer Temperatur von mindestens 72°C für etwa 15 bis 30 Sekunden, und mittels einer (optionalen) zweiten Pumpe 8 durch eine zweite Rohrleitung 9 zu einer Herstellungsvorrichtung 10 zum Herstellen des zumindest einen Teils des Fasern umfassenden Behälters transportiert. In der Herstellungsvorrichtung 10 kann das sterilisierte Rohmaterial beispielsweise in eine Form eingebracht werden, deren Innenfläche, zumindest im Wesentlichen, einer Negativform des herzustellenden zumindest einen Teils des Fasern umfassenden Behälters entsprechen kann.

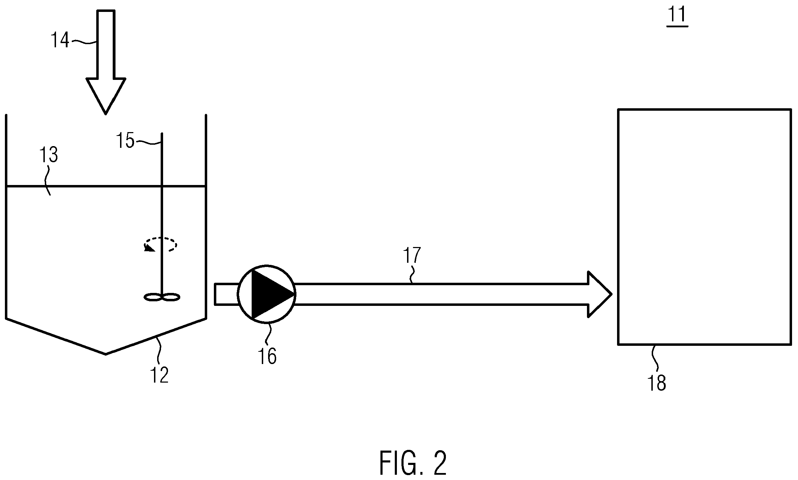

Die Figur 2 zeigt eine zweite Ausführungsform einer Vorrichtung 11 zum Sterilisieren von Rohmaterial 13 zum Herstellen zumindest eines Teils eines Fasern umfassenden Behälters. Das zu sterilisierende Rohmaterial 13 wird in einem Ausmischbehälter 12 bereitgestellt, wobei dem Rohmaterial 13 in dem Ausmischbehälter 12 mittels einer Zugabevorrichtung 14 zum Zugeben von chemischem Desinfektionsmittel ein chemisches Desinfektionsmittel zugesetzt werden kann, um ein chemisches Sterilisieren durchführen zu können. Das chemische Desinfektionsmittel kann H₂O₂, O₂, O₃, NaClO₂, NaClO, HClO, HOCI-Wasser oder ein elektrochemisch aktiviertes Wasser umfassen. Die Mischung aus Rohmaterial 13 und chemischem Desinfektionsmittel kann mittels eines Rührers 15 dispergiert werden kann. Beim chemischen Sterilisieren kann das Rohmaterial somit direkt im Ausmischbehälter 12 sterilisiert werden. Das chemische Desinfektionsmittel kann einer Dispergierflüssigkeit in entsprechender Konzentration zugegeben werden und je nach Konzentration und Einwirkzeit wirken. Das sterilisierte Rohmaterial wird mittels einer Pumpe 16 durch eine Rohrleitung 17 zu einer Herstellungsvorrichtung 18 zum Herstellen des zumindest einen Teils des Fasern umfassenden Behälters transportiert. In der Herstellungsvorrichtung 18 kann das sterilisierte Rohmaterial beispielsweise in eine Form eingebracht werden, deren Innenfläche, zumindest im Wesentlichen, einer Negativform des herzustellenden zumindest einen Teils des Fasern umfassenden Behälters entsprechen kann.

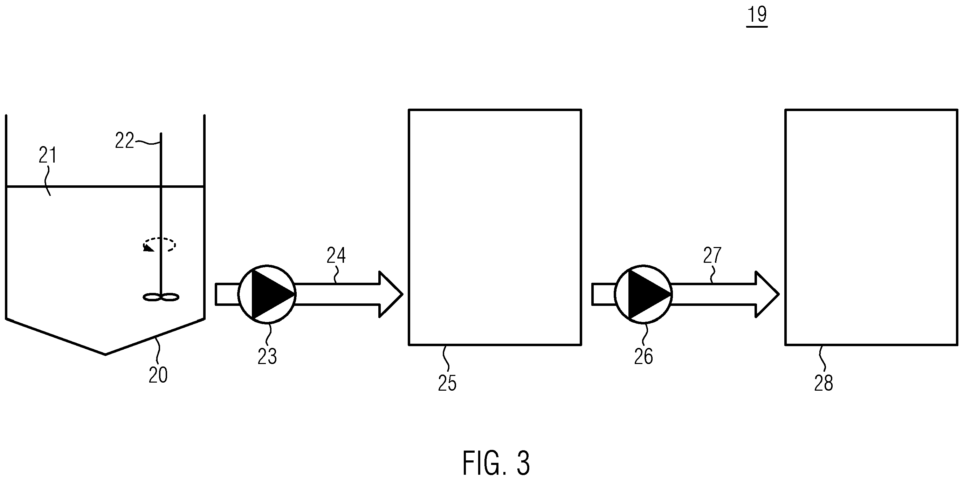

Die Figur 3 zeigt eine dritte Ausführungsform einer Vorrichtung 19 zum Sterilisieren von Rohmaterial 21 zum Herstellen zumindest eines Teils eines Fasern umfassenden Behälters. Das zu sterilisierende Rohmaterial 21 wird in einem Ausmischbehälter 20 bereitgestellt, in dem es mittels eines Rührers 22 dispergiert werden kann. Danach wird das Rohmaterial 21 mittels einer ersten Pumpe 23 durch eine erste Rohrleitung 24 zu und durch einen physikalischen Sterilisator 25 transportiert. Hier ist der physikalische Sterilisator 25 als Aggregat zwischen den Rohrleitungen 24, 27 ausgebildet. Es kann aber auch vorgesehen sein, dass die physikalische Sterilisierung in den Rohrleitungen erfolgen kann, sodass kein zusätzliches Aggregat erforderlich ist. Das physikalische Sterilisieren kann mittels Mikrowellenstrahlung, mittels eines Ohmschen Erhitzers, mittels Ultraviolettstrahlung, mittels einer Hochfrequenzerhitzung, mittels eines Pulsed Electric Field, mittels Plasma und/oder mittels Hochdruck erfolgen. In dem physikalischen Sterilisator 25 wird das Rohmaterial 21 zu sterilisiertem Rohmaterial sterilisiert und mittels einer optionalen zweiten Pumpe 26 durch eine zweite Rohrleitung 27 zu einer Herstellungsvorrichtung 28 zum Herstellen des zumindest einen Teils des Fasern umfassenden Behälters transportiert. In der Herstellungsvorrichtung 28 kann das sterilisierte Rohmaterial beispielsweise in eine Form eingebracht werden, deren Innenfläche, zumindest im Wesentlichen, einer Negativform des herzustellenden zumindest einen Teils des Fasern umfassenden Behälters entsprechen kann.

Trockenes Rohmaterial kann z.B. jeweils mit einem nicht gezeigten Gebläse transportiert werden.

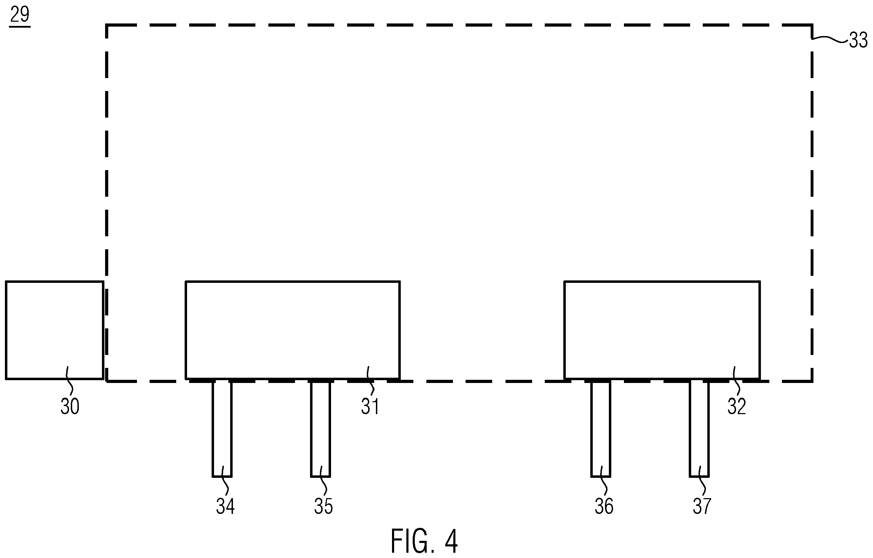

Die Figur 4 zeigt eine Behälterbehandlungsanlage 29, die eine Bereitstellungsvorrichtung 30 zum Bereitstellen einer sterilen, Fasern umfassenden Pulpe, eine Herstellungsvorrichtung 31 zum Herstellen eines die Fasern umfassenden Behälters aus der sterilen, Fasern umfassenden Pulpe und eine Füll- und Verschließvorrichtung 32 zum Füllen des sterilen, Fasern umfassenden Behälters mit Produkt und zum Verschließen des befüllten sterilen, Fasern umfassenden Behälters umfasst. Von der Bereitstellungsvorrichtung 30 bis zu der Füll- und Verschließvorrichtung 32 erstreckt sich ein aseptischer Raum 33, der ausgebildet ist, eine definierte aseptische Umgebungsbedingung für den sterilen, Fasern umfassenden Behälter aufrecht zu erhalten.

Die sterile, Fasern umfassende Pulpe kann mit den bereits zuvor beschriebenen Verfahrensschritten hergestellt und dann durch die Bereitstellungsvorrichtung 30 für das Herstellen der Fasren umfassenden Behälter zu Verfügung gestellt werden. Um die Sterilität der Fasern umfassenden Pulpe und dann auch des daraus hergestellten Fasern umfassenden Behälters gewährleisten zu können, sind die Maschinen von der Bereitstellungsvorrichtung 30 bis zu der Füll- und Verschließvorrichtung 32 im aseptischen Raum 33 angeordnet.

Beispielsweise können die Form (Kavität), die zur Herstellung eines Fasern umfassenden Behälters verwendet werden kann, steril sein, um eine Kontamination der sterilen, Fasern umfassenden Pulpe, die während eines Herstellungsprozesses in die Form eingebracht werden kann, zu vermeiden. Nach dem Herstellen kann der Fasern umfassende Behälter mittels der Füll- und Verschließvorrichtung 32 mit Produkt befüllt und anschließend verschlossen werden, sodass auch das Füllen und Verschließen unter den definierten aseptische Umgebungsbedingung erfolgen.

Im aseptischen Raum sind die Herstellungsvorrichtung 31 und die Füll- und Verschließvorrichtung 32 angeordnet, wobei jedoch Antriebe der Herstellungsvorrichtung 31 und der Füll- und Verschließvorrichtung 32 außerhalb des aseptischen Raums 33 angeordnet sind. Abgesehen von den Antrieben können die Elemente der Herstellungsvorrichtung 31 und der Füll- und Verschließvorrichtung 32 den definierte aseptische Umgebungsbedingung genügen.

Die Herstellungsvorrichtung 31 umfasst hier beispielhaft einen Antrieb 34 für ein Öffnen und Schließen der Form und einen Antrieb 35 für ein Anheben oder Senken eines Fasern umfassenden Behälters. Die Füll- und Verschließvorrichtung 32 umfasst hier beispielhaft einen Antrieb 36 für eine Bewegung von Fülldüsen und einen Antrieb 37 für eine Bewegung von Verschließköpfen.

## Patentansprüche

1. Verfahren zum Sterilisieren von Rohmaterial (3, 13, 21) zum Herstellen zumindest eines Teils eines Fasern umfassenden Behälters, wobei das Verfahren umfasst:
- Bereitstellen des Rohmaterials (3, 13, 21),
- Sterilisieren des Rohmaterials (3, 13, 21) zu sterilisiertem Rohmaterial.

2. Das Verfahren nach Anspruch 1, wobei das Sterilisieren ein physikalisches und/oder elektrothermisches Sterilisieren umfasst, wobei beispielsweise das physikalische Sterilisieren mittels eines physikalischen Sterilisators (25) erfolgt, wie mittels Mikrowellenstrahlung, mittels eines Ohmschen Erhitzers, mittels Ultraviolettstrahlung, mittels einer Hochfrequenzerhitzung, mittels eines Pulsed Electric Field, mittels Plasma und/oder mittels Hochdruck.

3. Das Verfahren nach Anspruch 1 oder 2, wobei das Sterilisieren eine thermische Erhitzung umfasst, wobei beispielsweise die thermische Erhitzung mittels einer Kurzeiterhitzungsvorrichtung (7) erfolgt.

4. Das Verfahren nach einem der Ansprüche 1 bis 3, wobei das Sterilisieren ein chemisches Sterilisieren umfasst, wobei beispielsweise das chemische Sterilisieren mittels einer Zugabe von chemischem Desinfektionsmittel erfolgt, wobei beispielsweise das chemische Desinfektionsmittel H₂O₂, O₂, O₃, NaClO₂, NaClO, HClO, HOCI-Wasser oder elektrochemische aktiviertes Wasser umfasst.

5. Das Verfahren nach einem der Ansprüche 1 bis 4, wobei das Rohmaterial (3, 13, 21) Fasern umfasst, beispielsweise trockene Fasern, wobei beispielsweise die Fasern Naturfasern umfassen.

6. Das Verfahren nach einem der Ansprüche 1 bis 5, wobei das Rohmaterial (3, 13, 21) Fasern und ein Fluid umfasst, wobei beispielsweise die Fasern Naturfasern umfassen, wobei beispielsweise das Fluid Wasser umfasst, wobei beispielsweise das Fluid Additive umfasst.

7. Sterilisiertes Rohmaterial zum Herstellen zumindest eines Teils eines Fasern umfassenden Behälters, wobei das sterilisierte Rohmaterial mittels des Verfahrens nach einem der Ansprüche 1 bis 6 erhalten wurde.

8. Verfahren zum Herstellen eines Fasern umfassenden Behälters unter Verwendung des sterilisierten Rohmaterials nach Anspruch 7.

9. Fasern umfassender Behälter, wobei zumindest ein Teil des Fasern umfassenden Behälters sterilisiertes Rohmaterial umfasst, das mittels des Verfahrens nach einem der Ansprüche 1 bis 6 erhalten wurde.

10. Der Fasern umfassende Behälter nach Anspruch 8, wobei der Fasern umfassende Behälter mittels des Verfahrens nach Anspruch 8 hergestellt wurde.

11. Vorrichtung zum Sterilisieren von Rohmaterial zum Herstellen zumindest eines Teils eines Fasern umfassenden Behälters, wobei die Vorrichtung ausgebildet ist, das Verfahren nach einem der Ansprüche 1 bis 6 auszuführen.

12. Die Vorrichtung nach Anspruch 11, weiter umfassend eine Zuführvorrichtung für Rohmaterial (3, 13, 21) und eine Sterilisiervorrichtung (7, 14, 25) zum Sterilisieren des Rohmaterials.

13. Die Vorrichtung nach Anspruch 12, wobei die Sterilisiervorrichtung (7, 14, 25) mindestens eines der Folgenden umfasst:
- eine Vorrichtung zur Erzeugung von Mikrowellenstrahlung,
- einen Ohmschen Erhitzer,
- eine Vorrichtung zur Induktionserhitzung, - eine Vorrichtung zur Erzeugung von Ultraviolettstrahlung,
- eine Vorrichtung zur Erzeugung von Infrarotlicht,
- eine Vorrichtung zur Erzeugung einer Hochfrequenz,
- eine Vorrichtung zur Erzeugung eines Pulsed Electric Field,
- eine Vorrichtung zur Erzeugung von Plasma,
- eine Vorrichtung zur Erzeugung von Hochdruck,
- eine Kurzeiterhitzungsvorrichtung,
- eine Zugabevorrichtung zum Zugeben von chemischem Desinfektionsmittel.

14. Behälterbehandlungsanlage (29) umfassend eine Bereitstellungsvorrichtung (30) zum Bereitstellen einer sterilen, Fasern umfassenden Pulpe, eine Herstellungsvorrichtung (31) zum Herstellen eines die Fasern umfassenden Behälters aus der sterilen, Fasern umfassenden Pulpe und eine Füll- und Verschließvorrichtung (32) zum Füllen des sterilen, Fasern umfassenden Behälters mit Produkt und zum Verschließen des befüllten sterilen, Fasern umfassenden Behälters und mindestens einen aseptischen Raum (33) umfasst, der ausgebildet ist, eine definierte aseptische Umgebungsbedingung für den sterilen, Fasern umfassenden Behälter aufrecht zu erhalten, und der sich von der Bereitstellungsvorrichtung (30) bis zu der Füll- und Verschließvorrichtung (32) erstreckt.

15. Die Behälterbehandlungsanlage nach Anspruch 14 wobei der mindestens eine aseptische Raum (33) ein Raum mit kontaminationsarmer Umgebung ist,
wobei beispielsweise die kontaminationsarme Umgebung ein Reinraum ist,
wobei beispielsweise die kontaminationsarme Umgebung durch eine oder Kombinationen der folgenden Maßnahmen erhalten wird:
- die kontaminationsarme Umgebung wird unter einen Überdruck im Vergleich zur Umgebungsatmosphäre gesetzt, beispielsweise durch das Vorsehen von Feinfiltem zum Einblasen von durch die Feinfilter filtrierte Luft zumindest während eines Betriebsmodus' der Behälterbehandlungsanlage in den aseptischen Raum (33),
- Anordnen von Antrieben (34, 35, 36, 37) für verschiedenste Bewegungen einer Form zum Herstellen des Fasern umfassenden Behälters und/oder von Transportelementen außerhalb des aseptischen Raums (33),
- Reinigung und/oder Sterilisation einer Oberfläche einer inneren Wandung des aseptischen Raums in regelmäßigen Abständen, beispielsweise Reinigung und/oder Sterilisation von Innen- und Außenseiten der Form und/oder der Fülldüsen und/oder von Verschließköpfen, beispielsweise Reinigung und/oder Sterilisation von Oberflächen von inneren Wandungen des Maschinenschutzes,
- Abdichtung der kontaminationsarme Umgebung gegen die Umgebungsatmosphäre, beispielsweise durch Vorsehen eines Wasserschlosses oder einer Gummidichtung als Dichtung bei einem Rundläufer zum Abdichten des drehenden Teils zum stehenden Teil des Rundläufers.
